# EUROPÄISCHE PATENTSCHRIFT

(11) **EP 1 504 783 B1**
(45) Veröffentlichungstag und Bekanntmachung des Hinweises auf die Patenterteilung: **05.09.2007**
(21) Anmeldenummer: 04017563.0
(22) Anmeldetag: 24.07.2004
(51) Int. Cl.: A61M 15/08, B65D 81/32, B05B 11/00

(54) **Dosiervorrichtung mit Applikationskuppe**
Dosing device with a nozzle
Dispositif de dosage pourvu d'une saillie

(30) Priorität: 01.08.2003 DE 10336293
(43) Veröffentlichungstag der Anmeldung: 09.02.2005
(73) Patentinhaber: Ing. Erich Pfeiffer GmbH, 78315 Radolfzell (DE)
(72) Erfinder: Tempfli, Karl, 78244 Gottmadingen (DE)
(74) Vertreter: Ruff, Michael

(56) Entgegenhaltungen:
- WO-A-02/068029
- WO-A-03/000310
- WO-A-03/006320
- DE-A- 10 110 742
- DE-A- 19 940 236
- DE-A- 19 963 946
- GB-A- 1 103 534

## Beschreibung

Die Erfindung betrifft eine Dosiervorrichtung mit einer Nasenolive, in der wenigstens eine Applikationsöffnung vorgesehen ist, sowie mit einer Zuführvorrichtung, die in Funktion ein erstes Medium durch wenigstens einen Mediumkanal aus einem Mediumspeicher zu der Applikationsöffnung führt.

Derartige Dosiervorrichtungen sind aus dem Stand der Technik, siehe z.B. die Patentschrift WO-A-03/000310, in vielfältigen Ausführungen bekannt. Sie werden insbesondere zur Dosierung von flüssigen, gasförmigen oder pulverförmigen Medien eingesetzt und erlauben insbesondere die Zufuhr derartiger Medien in Körperöffnungen für medizinische und therapeutische Zwecke. Dazu ist an der Dosiervorrichtung eine Applikationskuppe vorgesehen, die eine insbesondere schlanke und in einem stirnseitigen Endbereich abgerundete Kontur aufweist. Die Applikationskuppe ist mit der abgerundeten Kontur in eine Körperöffnung einführbar. In der Applikationskuppe ist wenigstens eine Applikationsöffnung vorgesehen, durch die das zu dosierende, druckbeaufschlagte Medium in eine Umgebung abgegeben werden kann. Eine Applikationsöffnung kann insbesondere als Bohrung mit einem auf das Medium abgestimmten Bohrungsquerschnitt in der Applikationskuppe vorgesehen sein. Über den Bohrungsquerschnitt wird eine Dosierweise des Mediums stark mitbeeinflusst, da sich das druckbeaufschlagte Medium beim Austritt aus der Applikationsöffnung nahezu schlagartig auf einen Umgebungsdruck entspannt. Abhängig von einem Druckgradienten im Bohrungsquerschnitt sowie einer Düsenwirkung durch den Bohrungsquerschnitt wird das Medium bei der Dosierung stärker oder schwächer entspannt und davon abhängig zerstäubt. Als Bohrungsquerschnitt kommt insbesondere ein zylindrischer, konischer, hyperbolischer oder sphärischer Querschnitt oder eine Kombination davon in Frage.

Das Medium wird von einer Zuführvorrichtung mit Druck beaufschlagt und durch die Applikationsöffnung gefördert, dabei ist die Zuführvorrichtung insbesondere als Druckspeicher oder Druckerzeugungsvorrichtung ausgeführt. Bei einem Druckspeicher ist das Medium mit einem statischen Überdruck beaufschlagt, der ein Ausströmen des Mediums durch die Applikationsöffnung in die Umgebung ermöglicht. Eine Druckerzeugungsvorrichtung kann insbesondere als manuell betätigbare Pumpvorrichtung ausgeführt sein, bei der das Medium durch Bedienkräfte eines Benutzers druckbeaufschlagt wird und somit durch die Applikationsöffnung in die Umgebung ausgebracht wird.

Das Medium ist in einem Mediumspeicher bevorratet, der entweder der Dosiervorrichtung fest zugeordnet ist oder als separater Bestandteil hinzufügbar vorgesehen ist. Zum Transport des Mediums zur der Applikationsöffnung ist in der Applikationskuppe ein Mediumkanal vorgesehen, der von dem Mediumspeicher zu der Applikationsöffnung führt. Der Mediumkanal kann dabei insbesondere einstückig mit der Applikationskuppe verbunden sein. Eine separate Ausführung des Mediumkanals mit einem Befestigungsbereich gegenüber der Applikationskuppe ist ebenfalls vorstellbar. Das Medium wird in Funktion der Zuführvorrichtung aus dem Mediumspeicher zu der Applikationsöffnung geführt und dort bedingt durch eine Druckdifferenz zwischen dem Medium und der Umgebung insbesondere versprüht, zerstäubt oder abgestrahlt.

Die Aufgabe der Erfindung besteht darin, eine Dosiervorrichtung der eingangs genannten Art zu schaffen, bei der eine verbesserte Dosierung erreicht wird.

Diese Aufgabe wird dadurch gelöst, dass mehrere Ausströmöffnungen zirkular um die Applikationsöffnung herum in der Nasenolive angeordnet sind, und dass innerhalb der Nasenolive wenigstens ein Strömungskanal für das wenigstens eine weitere Medium zu den Ausströmöffnungen geführt ist. Eine Ausströmöffnung kann insbesondere wie eine Applikationsöffnung gestaltet sein, weiterhin ist auch eine beliebige Profilierung, wie insbesondere als Kreisringabschnitt, Kreissegment, Stern- oder Ovalöffnung möglich. Das wenigstens eine weitere Medium kann insbesondere flüssig, gasförmig, pulverförmig oder als Gemisch von Medien in unterschiedlichen Aggregatszuständen vorgesehen sein. Die Ausströmöffnungen für das wenigstens eine weitere Medium sind in der Nasenolive benachbart zu der Applikationsöffnung angeordnet. Dabei kann eine Ausströmrichtung sowie eine Ausströmkontur für das wenigstens eine weitere Medium derart auf das erste Medium abgestimmt sein, dass das erste Medium durch das wenigstens eine weitere Medium insbesondere beschleunigt, abgelenkt, ummantelt oder verteilt wird. Innerhalb der Nasenolive ist dafür wenigstens ein Strömungskanal vorgesehen, der das wenigstens eine weitere Medium zu der Ausströmöffnung führt und somit insbesondere ein synchrones Ausströmen des ersten Mediums und des wenigstens einen weiteren Mediums ermöglicht. Der Strömungskanal kann dabei als separates Bauteil in der Nasenolive vorgesehen sein oder durch geeignete Anordnung des Mediumkanals in der Nasenolive begrenzt werden. Vorzugsweise ist als weiteres Medium Luft vorgesehen, das ein flüssiges, erstes Medium bei der Applikation in die Nase unterstützt.

In Ausgestaltung der Erfindung sind die Ausströmöffnungen an einem stirnseitigen Endbereich der Nasenolive vorgesehen. Für eine Verwendung der erfindungsgemäßen Dosiervorrichtung in Körperöffnungen ist insbesondere vorzusehen, dass sowohl das zu dosierende erste Medium als auch das wenigstens eine, weitere Medium, insbesondere geradlinig oder kegelförmig in Richtung einer Hauptachse der Applikationskuppe ausgebracht werden. Durch die Anordnung der Ausströmöffnungen an einem stirnseitigen Endbereich der Applikationskuppe kann eine derartige Ausströmung der Medien sichergestellt werden. In einer bevorzugten Ausführungsform sind die Ausströmungsrichtungen der Ausströmöffnungen symmetrisch zu einer Applikationsrichtung der Applikationsöffnung vorgesehen.

In weiterer Ausgestaltung der Erfindung sind die Ausströmöffnungen konzentrisch zur Applikationsöffnung vorgesehen. Bei einer konzentrischen Anordnung der Ausströmöffnungen zur Applikationsöffnung ergibt sich eine besonders vorteilhafte Wechselwirkung zwischen dem ersten Medium und dem zumindest einen weiteren Medium während des Dosiervorgangs, da das wenigstens eine weitere Medium das erste Medium zumindest teilweise mantelförmig umschließt und somit eine Führungsfunktion für das erste Medium ausüben kann. Die konzentrisch zur Applikationsöffnung vorgesehenen Ausströmöffnungen können insbesondere kreisringförmig oder abschnittsweise kreisringförmig mit einem rechteckigen, konischen, hyperboloiden oder sphärischen Querschnitt oder als Kombination davon ausgeführt sein.

In der Dosiervorrichtung gemäß der Erfindung sind mehrere Ausströmöffnungen zirkular um die Applikationsöffnung herum angeordnet. Eine zirkulare Anordnung der Ausströmöffnungen bedeutet insbesondere eine Nutzung von Vorteilen einer konzentrischen Anordnung mit der zusätzlichen Möglichkeit, durch unterschiedliche Querschnitte und/oder Profile der Ausströmöffnungen eine Strahlformung durch Wechselwirkung der austretenden Medien zu erzielen. Dabei können die Ausströmöffnungen auf einem oder mehreren konzentrisch zur Applikationsöffnung angeordneten Kreisen oder Kreisabschnitten vorgesehen werden, wobei die Ausströmrichtungen auf die gewünschte Ausströmkontur des ersten Mediums abgestimmt werden können. In einer bevorzugten Ausführungsform ist eine Mehrzahl von Ausströmöffnungen auf einem einzigen konzentrisch zur Applikationsöffnung angeordneten Kreis vorgesehen, alle Ausströmöffnungen weisen dabei auch einen identischen Querschnitt sowie eine identische Ausströmrichtung auf.

Weitere Vorteile und Merkmale der Erfindung ergeben sich aus den Ansprüchen sowie aus der nachfolgenden Beschreibung eines bevorzugten Ausführungsbeispiels, das anhand der Zeichnungen dargestellt ist.

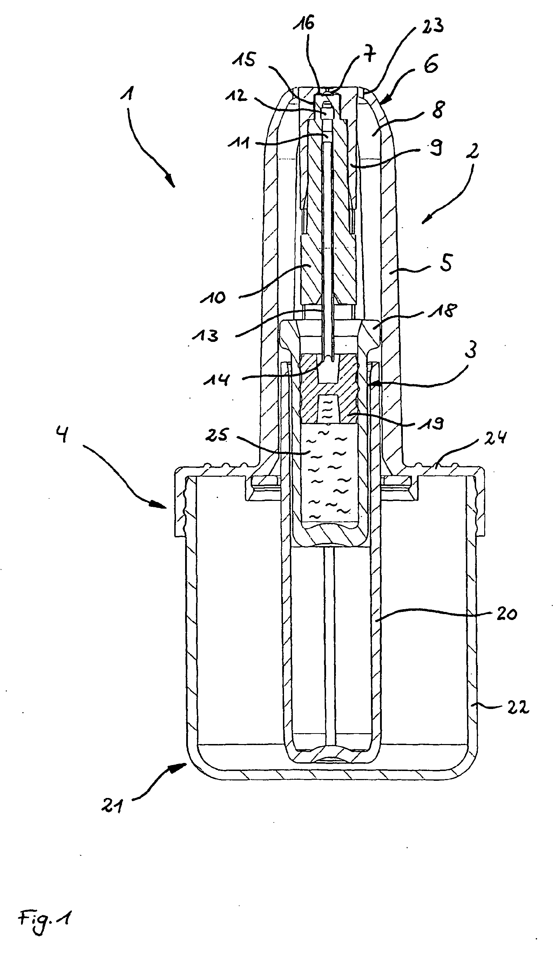

- Fig. 1: zeigt in ebener Schnittdarstellung eine Dosiervorrichtung mit Applikationskuppe, Zuführvorrichtung und Mediumspeicher, und

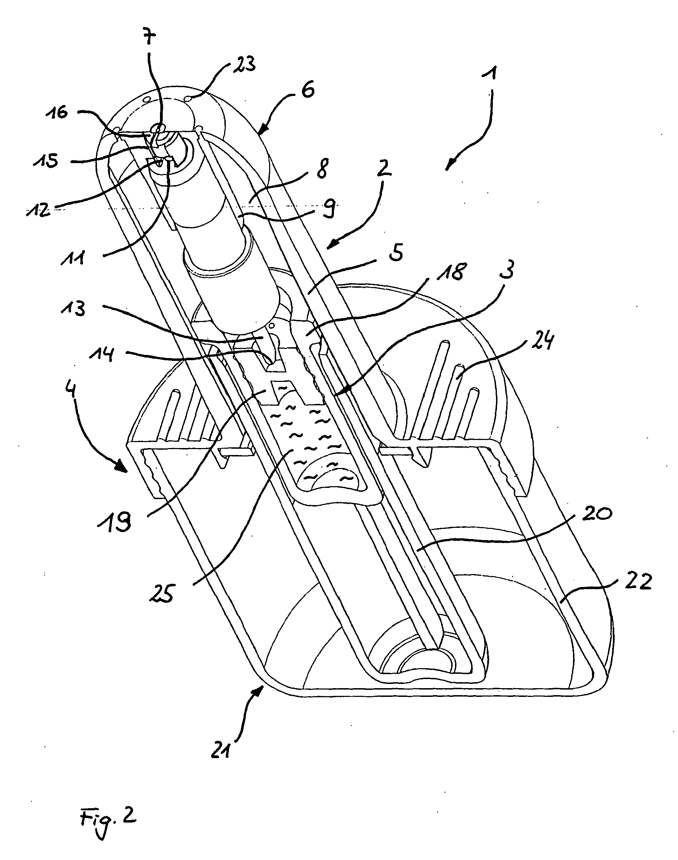

- Fig. 2: in isometrischer Schnittdarstellung eine Dosiervorrichtung gemäß Fig. 1.

Eine Dosiervorrichtung 1 weist einen Applikator 2, eine als Zuführeinrichtung ausgeführte, manuell betätigbare Dosierpumpe 3 sowie ein Griffteil 4 auf. Die Dosierpumpe 3 ist für eine Einmalbetätigung ausgeführt, d.h. sie wird für eine einmalige Applikation benötigt. Es wird lediglich ein einmaliger Pumphub durchgeführt. Der Applikator 2 weist eine als Applikationskuppe ausgeführte Nasenolive 5 auf, die als schlanke Hülse ausgeführt ist. In einem stirnseitig verschlossenen Endbereich 6 weist die Nasenolive 5 eine Applikationsöffnung 7 auf, die rotationssymmetrisch zu einer Mittellängsachse der Nasenolive 5 ausgeführt ist. Vom Endbereich 6 ragt eine Führungsmuffe 9 in einen von der Nasenolive 5 umschlossenen Innenraum 8. Die Führungsmuffe 9 ist rotationssymmetrisch ausgeführt und weist eine im wesentlichen zylindrische Außenkontur sowie eine zylindrisch gestufte Innenkontur auf, die in einem Endbereich zur Aufnahme eines Füllstücks 10 konisch ausgestaltet ist. Das Füllstück 10 ist kraftschlüssig in die Führungsmuffe 9 eingepresst und weist eine konzentrisch zur Mittellängsachse angeordnete Innenbohrung 11 sowie eine orthogonal zur Mittellängsachse angeordnete Querbohrung 12 auf. In die Innenbohrung 11 ist ein Rohrabschnitt 13 formschlüssig eingepresst, der an einem der Applikationsöffnung abgewandten Ende eine schräge Schneidkante 14 aufweist. Der Rohrabschnitt 13 bildet zusammen mit der Innenbohrung 11 und der Querbohrung 12 einen Abschnitt eines Mediumkanals, der sich von der Schneidkante 14 bis zur Applikationsöffnung 7 erstreckt.

In einem Bereich zwischen der Querbohrung 12 und der Applikationsöffnung 7 wird der Mediumkanal aus einem Zylinderspalt 15 sowie einer Dichttellerfläche 16 gebildet, die durch eine Beabstandung der Führungsmuffe 9 vom Füllstück 10 verwirklicht sind. Während Führungsmuffe 9 und Füllstück 10 im Bereich der Dichttellerfläche 16 in einer Ruhestellung zumindest abschnittsweise flächig abdichtend aufeinanderliegen, wird eine Stirnfläche der Führungsmuffe 9, die auch die Applikationsöffnung 7 enthält, bei einem steigenden Mediumdruck im Mediumkanal deformiert. Dadurch wird die Dichtwirkung zwischen Führungsmuffe 9 und Füllstück 10 zeitweilig aufgehoben und das Medium kann durch den Mediumkanal und die Applikationsöffnung 7 in eine Umgebung abgegeben werden kann.

Der Innenraum 8 der Nasenolive 5 ist im Endbereich 6 durch als Axialbohrungen 23 ausgeführte Ausströmöffnungen kommunizierend mit der Umgebung verbunden. Durch die Anordnung der Führungsmuffe 9 und des Füllstücks 10 in der Nasenolive 5 ist der Innenraum 8 zumindest abschnittsweise als Strömungskanal für zumindest ein weiteres Medium vorgesehen. An einem der Applikationsöffnung 7 abgewandten Ende des Füllstücks 10 ist ein im Bereich der Nasenolive 5 geführter Mediumspeicher 18 vorgesehen, der im Ruhezustand durch einen Dichtstopfen 19 verschlossen ist. Der Mediumspeicher 18 ist zumindest abschnittsweise von einer Druckhülse 20 umgeben, die ebenfalls abschnittsweise in der Nasenolive 5 geführt ist. Für eine Kraftübertragung zwischen der Druckhülse 20 und dem Mediumspeicher 18 ist am Mediumspeicher 18 ein umlaufender Bund vorgesehen, mit dem eine Stirnfläche der Druckhülse 20 formschlüssig in Kontakt treten kann. Die Druckhülse 20 wird an einem vom Endbereich 6 der Nasenolive 5 abgewandten Betätigungsbereich 21 von einem flexiblen Druckbalg 22 umgeben, der mit dem Applikator 2 eine Außenkontur der Dosiervorrichtung 1 bestimmt. Der Druckbalg 22 schließt ein Luftvolumen ein, das bei einem Dosiervorgang durch den als Strömungskanal ausgeführten Innenraum 8 der Nasenolive 5 durch die Ausströmöffnungen 23 in die Umgebung abgegeben werden kann.

Für einen Dosiervorgang der Dosiervorrichtung 1 legt ein Benutzer zumindest zwei Finger, insbesondere den Mittel- und den Zeigefinger auf eine Auflagefläche 24 des Applikators 2 auf. Mit einem weiteren Finger, insbesondere dem Daumen, wird eine Betätigungskraft auf den Betätigungsbereich 21 ausgeübt. Durch die Betätigungskraft wird der Druckbalg 22 deformiert, bis er in Anlage zur Druckhülse 20 kommt. Anschließend wird die Druckhülse 20 in Richtung des Endbereichs 6 verschoben, bis sie an einem vorderen Stirnende formschlüssig mit dem umlaufenden Bund des Mediumspeichers 18 in Kontakt tritt. Daraufhin wird die Druckhülse 20 synchron mit dem Mediumspeicher 18 weiter in Richtung des Endbereichs 6 verschoben, bis die Schneidkante 14 des Rohrabschnitts 13 auf eine als Durchstechmembran ausgeführte Oberfläche des Dichtstopfens 19 auftrifft. Durch die Abschrägung der Schneidkante 14 des Rohrabschnitts 13 kann bei entsprechender Betätigungskraft die Durchstechmembran des Dichtstopfens 19 durchstoßen werden, wodurch ein im Mediumspeicher eingeschlossenes Medium 25 kommunizierend mit dem Mediumkanal in Verbindung tritt.

Durch weitere Ausübung der Betätigungskraft auf den Betätigungsbereich 21 der Dosiervorrichtung 1 werden die Druckhülse 20 und der Mediumspeicher 18 weiter in Richtung des Endbereichs 6 verschoben, wobei im Mediumspeicher ein Druckaufbau stattfindet, sobald eine Stirnseite des Füllstücks 10 auf den als Pumpkolben ausgeführten Dichtstopfen 19 aufläuft und diesen in Richtung des eingeschlossenen Mediums 25 verschiebt. Dadurch wird das Medium 25 in den Mediumkanal gepresst und strömt entlang des Rohrabschnitts 13 in die Innenbohrung 11 und anschließend in die Querbohrung 12 sowie in den daran anschließenden Zylinderspalt 15. Sobald ein entsprechender Druckaufbau stattgefunden hat, deformiert sich die Führungsmuffe 9, so dass die Dichttellerfläche 16 aus ihrer Abdichtposition in eine Strömungsposition übergeht und ein Strömen des Mediums durch die Applikationsöffnung 7 ermöglicht. Durch die Geometrie der Applikationsöffnung 7 und den Überdruck des Mediums findet eine Medienzerstäubung statt, bei der feine Mediumpartikel mit einer druckabhängigen Austrittsgeschwindigkeit von der Dosiervorrichtung 1 in die Umgebung abgegeben werden. Da es gleichzeitig und synchron zur Bewegung der Druckhülse 20 auch zu einer Deformation des Druckbalgs 22 kommt, strömt zeitgleich zum Medium die vom Druckbalg 22 eingeschlossene Luft längs des Innenraums 8 der Nasenolive 5 durch die Ausströmöffnungen 23 in die Umgebung und bildet einen Mantelstrom für das Medium, das aus dem Mediumspeicher 18 ausgebracht wird. Der Mantelstrom sorgt insbesondere für eine größere Reichweite der Dosierung in der Körperöffnung, so dass für therapeutische Anwendungen eine geringere Mediummenge vorgesehen werden kann, um den gleichen Erfolg wie bei Dosiervorrichtungen aus dem Stand der Technik zu erzielen.

In einer weiteren, nicht dargestellten Ausführungsform der Erfindung ist anstelle der Einmal-Pumpe ein Mehrfachpumpsystem als Zuführeinrichtung vorgesehen. Bei einem Mehrfachpumpsystem wird ein Pumpkolben in bekannter Weise von einem Pumpstößel in einem Pumpgehäuse bewegt. Dadurch kann eine erste Mediummenge aus dem Mediumspeicher gefördert werden. Das zumindest eine weitere Medium kann durch ein parallel geschaltetes, weiteres Mehrfachpumpsystem ebenfalls aus einem Mediumspeicher gefördert werden. Bei Verwendung von Umgebungsluft als weiteres Medium kann auch eine Förderung durch das weitere Mehrfachpumpsystem unmittelbar aus der Umgebung vorgenommen werden.

In einer anderen, ebenfalls nicht dargestellten Ausführungsform ist zumindest ein Medium in einem druckbeaufschlagten Mediumspeicher bevorratet, wobei der Mediumspeicher durch eine Durchstechmembran oder ein Ventil verschlossen ist und zur Dosierung geöffnet werden kann. Ein druckbeaufschlagter Mediumspeicher kann insbesondere bei einem gasförmigen Medium durch komprimierte Speicherung des Mediums verwirklicht werden. Für flüssige Medien sind treibgasbefüllte Mediumspeicher bekannt sind.

In einer weiteren, nicht dargestellten Ausführungsform der Erfindung wird das zumindest eine weitere Medium durch eine Ausatembewegung eines Benutzers unter Druck gesetzt und der Dosiervorrichtung zugeführt. Dabei hält der Benutzer ein Mundstück mit den Lippen dicht umschlossen, so dass es bei der Ausatembewegung zu einem Überdruck am Mundstück kommt. Dieser Überdruck wird mittels eines Strömungskanals, der insbesondere als Schlauchleitung vorgesehen sein kann, in den Innenraum der Nasenolive zugeführt und bewirkt dadurch die gewünschte Umströmung des ersten Mediums durch die Ausströmöffnungen. Dabei ist vorzugsweise eine Synchronisation der Dosierung des ersten Mediums mit der Ausatembewegung druch Synchronisationsmittel vorzusehen.

## Patentansprüche

1. Dosiervorrichtung mit einer Nasenolive (5), in der wenigstens eine Applikationsöffnung (7) vorgesehen ist, sowie mit einer Zuführvorrichtung (3), die in Funktion ein erstes Medium (25) durch wenigstens einen Mediumkanal (11, 12, 13, 15, 16) aus einem Mediumspeicher (18) zu der Applikationsöffnung (7) führt, **dadurch gekennzeichnet, dass** die Nasenolive einstückig ausgebildet ist und dass mehrere Ausströmöffnungen (23) zirkular um die Applikationsöffnung (7) herum in der Nasenolive (5) angeordnet sind, und dass innerhalb der Nasenolive (5) wenigstens ein Strömungskanal (8) für das wenigstens eine weitere Medium zu den Ausströmöffnungen (23) geführt ist.

2. Dosiervorrichtung nach Anspruch 1, **dadurch gekennzeichnet, dass** die Ausströmöffnungen (23) an einem stirnseitigen Endbereich (6) der Nasenolive (5) vorgesehen sind.

3. Dosiervorrichtung nach Anspruch 1, **dadurch gekennzeichnet, dass** die Ausströmöffnungen (23) konzentrisch zur Applikationsöffnung (7) vorgesehen sind.

## Claims

1. Dosing device with a nose olive (5) containing at least one application opening (7), as well as with a supply device (3) which, when operating, guides a first medium (25) through at least one medium channel (11, 12, 13, 15, 16) from a medium reservoir (18) to the application opening (7), **characterized in that** the nose olive is constructed in one piece and that several discharge openings (23) are arranged in circular manner around the application opening (7) in nose olive (5) and that within said nose olive (5) at least one flow channel (8) for the at least one further medium leads to the discharge openings (23).

2. Dosing device according to claim 1, **characterized in that** the discharge openings (23) are provided on a front end region (6) of nose olive (5).

3. Dosing device according to claim 1, **characterized in that** the discharge openings (23) are provided concentrically to the application opening (7).

## Revendications

1. Dispositif de dosage comprenant une saillie (5) dans laquelle est prévue au moins une ouverture d'application (7), et un dispositif d'alimentation (3) qui, en fonctionnement, transporte un premier milieu (25) à travers au moins un canal de milieu (11, 12, 13, 15, 16) depuis un réservoir (18) vers l'ouverture d'application (7), **caractérisé en ce que** la saillie est configurée d'un seul tenant, **en ce que** plusieurs orifices d'émission (23) sont disposés de manière circulaire autour de l'orifice d'application (7) dans la saillie (5), et **en ce que** dans ladite saillie (5), au moins un canal d'écoulement (8) est prévu pour le milieu complémentaire au moins vers les orifices d'émission (23).

2. Dispositif de dosage selon la revendication 1, **caractérisé en ce que** les orifices d'émission (23) sont prévus dans une zone d'extrémité (6) avant de la saillie (5).

3. Dispositif de dosage selon la revendication 1, **caractérisé en ce que** les orifices d'émission (23) sont prévus de manière concentrique autour de l'orifice d'application (7).
